# FASCICULE DE BREVET EUROPEEN

(11) **EP 3 792 457 B1**
(45) Date de publication et mention de la délivrance du brevet: **02.11.2022**
(21) Numéro de dépôt: 20193804.0
(22) Date de dépôt: 01.09.2020
(51) Int. Cl.: F01M 1/10, F01M 11/10, F16N 29/04, F16N 39/06

(54) **PROCEDE ET SYSTEME DE SURVEILLANCE D'UN SYSTEME MECANIQUE LUBRIFIE**
VERFAHREN UND SYSTEM ZUR ÜBERWACHUNG EINES GESCHMIERTEN MECHANISCHEN SYSTEMS
METHOD AND SYSTEM FOR MONITORING A LUBRICATED MECHANICAL SYSTEM

(30) Priorité: 10.09.2019 FR 1909954
(43) Date de publication de la demande: 17.03.2021
(73) Titulaire: Airbus Helicopters, 13725 Marignane Cedex (FR)
(72) Inventeur: BELHABIB, Gilles, 13090 AIX EN PROVENCE (FR)
(74) Mandataire: GPI Brevets

(56) Documents cités:
- FR-A1- 2 508 168
- US-A1- 2002 148 788
- US-A1- 2013 332 045
- US-A1- 2017 248 572
- US-A1- 2019 162 687
- US-B1- 6 582 661
- Harkemanne Etienne ET AL: "Analysis and Testing of Debris Monitoring Sensors for Aircraft Lubrication Systems", Proceedings, vol. 2, no. 8, 15 July 2018 (2018-07-15), page 461, XP055887135, DOI: 10.3390/ICEM18-05360

## Description

La présente invention est du domaine des dispositifs mécaniques et des transmissions mécaniques et en particulier des transmissions mécaniques d'un aéronef.

La présente invention concerne un procédé et un système de surveillance d'un système mécanique lubrifié. Ce procédé et ce système de surveillance sont particulièrement adaptés à la surveillance du fonctionnement d'une boîte de transmission de puissance d'un aéronef à voilure tournante.

Un système mécanique comporte généralement des éléments mobiles, par exemple des éléments tournants, tels que des arbres et des roulements, ainsi que des éléments de transmission de puissance, voire de réduction ou d'augmentation de vitesse de rotation, tels que des pignons et/ou des engrenages. Il est alors essentiel pour le bon fonctionnement du système mécanique de lubrifier et de refroidir ces éléments tournants par un liquide de lubrification, par exemple de l'huile. Cette lubrification est en général assurée par un système de lubrification et a pour fonctions principales de limiter l'usure et l'échauffement de ces éléments tournants du système mécanique et, par suite, de prolonger leurs durées de vie. Sans une telle lubrification, le fonctionnement du système mécanique peut être rapidement dégradé, voire impossible.

Un système de lubrification comporte un réservoir stockant et récupérant le liquide de lubrification. De plus, le système de lubrification comporte un ou plusieurs circuits de lubrification et au moins un générateur de débit, par exemple une pompe, afin d'alimenter en liquide de lubrification chaque circuit de lubrification. Chaque circuit de lubrification peut comporter des gicleurs pulvérisant le liquide de lubrification sur au minimum les éléments tournants et de transmission mécanique essentiels du système mécanique.

Un circuit de lubrification peut aussi comporter au moins un dispositif de refroidissement, par exemple un échangeur thermique liquide/air, afin de limiter voire empêcher un échauffement du liquide de lubrification.

Par ailleurs, lors de son fonctionnement, le système mécanique peut générer des particules issues de ses différents éléments mobiles, par exemple au niveau des dentures des pignons et/ou des engrenages ainsi qu'au niveau des roulements. Ces particules peuvent provenir d'une usure normale de ces éléments tournants. Toutefois, ces particules peuvent également apparaître consécutivement à une dégradation d'au moins un de ces éléments tournants ou de transmission de puissance du système mécanique. Les éléments mobiles sont souvent métalliques, par exemple en acier ou en titane, Toutefois, des éléments mobiles peuvent également être non métalliques, par exemple en céramique.

De plus, des particules peuvent également être générées par des composants d'un circuit de lubrification, par exemple d'un dispositif de refroidissement.

Ces particules peuvent alors être entraînées dans le réservoir du système de lubrification et circuler dans chaque circuit de lubrification par l'intermédiaire du générateur de débit. Ces particules peuvent alors par exemple perturber le bon fonctionnement du générateur de débit et/ou obstruer un gicleur, perturbant ainsi la pulvérisation du liquide de lubrification effectuée par ce gicleur, voire la stoppant.

La présence de telles particules en quantité significative peut être un signe de la présence d'un dommage sur l'un des composants du système mécanique et d'un risque de défaillance prochaine de ce système mécanique.

Toutefois, ces particules peuvent aussi provenir d'une pollution du liquide de lubrification sans qu'il y ait de dégradation du système mécanique ou du système de lubrification. En outre, d'autres particules, métalliques ou non métalliques, peuvent également se trouver dans le réservoir ou circuler dans chaque circuit de lubrification.

Afin de limiter voire de stopper la circulation de particules métalliques ou non métalliques, un système de lubrification peut comporter un ou plusieurs dispositifs de captation permettant d'une part de filtrer le liquide de lubrification et d'autre part de capter certaines de ces particules.

On connait par exemple un ou plusieurs dispositifs de captation dite « mécanique » pouvant être intégrés au système de lubrification et permettant de bloquer certaines particules métalliques ou non métalliques. Des dispositifs de captation mécanique comportent des orifices afin de laisser passer le liquide de lubrification et bloquent de la sorte les particules de dimensions supérieures aux dimensions de ces orifices. De tels dispositifs de captation mécanique sont également communément dénommés « dispositifs de filtration ».

Un premier dispositif de captation mécanique est formé par exemple par une crépine et peut être agencé en amont d'un générateur de débit, au niveau d'une entrée d'aspiration d'un circuit de lubrification. Ce premier dispositif de filtration mécanique évite que certaines particules ne pénètrent dans le circuit de lubrification en les bloquant dans le réservoir.

Un deuxième dispositif de captation mécanique est formé par exemple par un filtre et peut être agencé dans un circuit de lubrification en amont et/ou en aval d'un dispositif de refroidissement du liquide de lubrification. Un deuxième dispositif de filtration mécanique agencé en amont du dispositif de refroidissement évite ainsi que certaines particules ne pénètrent dans le dispositif de refroidissement et s'accumulent éventuellement dans ce dispositif de refroidissement. Un deuxième dispositif de filtration mécanique agencé en aval du dispositif de refroidissement évite que des particules générées par ce dispositif de refroidissement ou bien préalablement piégées par ce dispositif de refroidissement se déplacent dans le circuit de lubrification.

Un troisième dispositif de captation mécanique est formé par exemple de nouveau par une crépine ou bien par un tamis et peut être agencé au niveau des gicleurs du système de lubrification afin de bloquer des particules ayant échappé aux précédents dispositifs de captation mécanique avant la pulvérisation du liquide de lubrification.

Un dispositif de captation peut également être magnétique et être agencé dans le système de lubrification afin de capter et de retenir uniquement les particules métalliques passant à proximité, contrairement à un dispositif de captation mécanique susceptible de capter toutes particules.

Par exemple, des dispositifs de captation magnétique se présentent sous la forme de bouchons magnétiques pouvant être agencés dans le réservoir d'un système de lubrification. Des bouchons magnétiques peuvent également être agencés dans une conduite d'un circuit de lubrification ou encore à l'entrée et/ou à la sortie d'un dispositif de refroidissement. Cependant, ces bouchons magnétiques ne peuvent capter qu'en partie les particules métalliques circulant à proximité, les autres particules métalliques pouvant circuler librement. En outre, les particules non métalliques ne sont pas captées par les bouchons magnétiques.

Un dispositif de captation peut aussi être formé par une zone de rétention des particules située dans le réservoir ou bien dans le flux du liquide de lubrification. Une zone de rétention des particules a une forme particulière permettant de piéger des particules, par exemple par gravité ou bien grâce à une force centrifuge générée par le flux du liquide de lubrification.

Par exemple, une zone de rétention peut comporter une cavité agencée au fond d'un réservoir, des particules présentes dans le liquide de lubrification s'accumulant par gravité dans cette cavité. Une zone de rétention peut aussi comporter une zone d'accumulation et une chicane agencée dans le flux du liquide de lubrification permettant de piéger dans la zone d'accumulation au moins en partie les particules circulant dans le liquide de lubrification.

Une zone de rétention peut aussi comporter une zone d'accumulation et une forme spécifique permettant la création d'un courant, par exemple un vortex, dans le liquide de lubrification. Le courant dans le liquide de lubrification permet alors d'orienter, par exemple grâce à la force centrifuge, au moins une partie des particules circulant dans le liquide de lubrification vers la zone d'accumulation où elles sont piégées.

Un bouchon éventuellement magnétique peut être agencé dans la cavité ou la zone d'accumulation de la zone de rétention afin de collecter les particules accumulées.

Ces divers dispositifs de captation peuvent être inspectés lors d'inspections de maintenances périodiques afin notamment de récupérer les particules captées. Une analyse de ces particules peut être effectuée afin de déterminer tout d'abord leur quantité, leur nature, notamment s'il s'agit de particules métalliques ou non métalliques, et éventuellement leurs origines, de sorte à définir si elles sont issues d'une usure normale ou bien d'une dégradation d'un élément mécanique du système mécanique. Une dégradation est par exemple une usure anormale et/ou inacceptable d'un élément mobile du système mécanique, une fissure sur un élément mobile, voire une cassure d'un élément mobile, Une dégradation nécessite généralement une intervention rapide afin de remplacer l'élément incriminé, le fonctionnement du système mécanique pouvant être dégradé voire rapidement stoppé.

Ces inspections programmées des dispositifs de captation représentent une charge de maintenance importante, ces dispositifs de captation pouvant être difficilement accessible. Toutefois, une dégradation intervenant entre deux inspections ne sera pas détectée avant l'inspection suivante et peut aboutir une dégradation importante, voire la destruction, du système mécanique.

Afin de pallier ce risque, des dispositifs de captation magnétique dit « à signalisation » ou « électrique » permettent d'une part de capter des particules métalliques et d'autre part de détecter qu'une quantité de particules métalliques supérieure à un seuil prédéterminé a été captée et de générer une alerte en conséquence. Toutefois, de tels dispositifs de captation magnétique à signalisation ne captent que les particules métalliques circulant à proximité de ces dispositifs de captation magnétique et nécessitent parfois de capter une quantité importante de particules métalliques pour déclencher une alerte. Dès lors, le système mécanique doit par précaution être immédiatement stoppé dès le déclenchement d'une alerte, ce qui provoque l'arrêt immédiat de la mission en cours et un atterrissage rapide dans le cadre d'un aéronef équipé de ce système mécanique potentiellement défaillant ainsi qu'une immobilisation de l'aéronef jusqu'à inspection, et éventuellement réparation ou changement, du système mécanique.

Le document FR 2927401 décrit un exemple de bouchon magnétique à signalisation équipant un système mécanique d'un aéronef.

Ces dispositifs sont intéressants. Toutefois, les particules métalliques captées peuvent provenir d'une pollution du liquide de lubrification sans qu'il y ait dégradation du système mécanique. En conséquence, l'arrêt immédiat d'un système mécanique peut être entrepris suite à une fausse alerte en présence d'une pollution du liquide de lubrification alors qu'en fait aucune dégradation mécanique n'est présente.

Un dispositif de détection de particules peut également être utilisé dans un système de lubrification. Un tel dispositif de détection peut être désigné par l'acronyme ODM pour la désignation en langue anglaise « Oil Débris Monitoring ». Un dispositif de détection ODM permet par exemple de compter les particules passant à proximité, et éventuellement de déterminer leurs tailles, leurs débits et/ou leurs masses. L'information fournie sur ces particules détectées peut donc être partielle, et les particules détectées continuent de circuler dans le circuit de lubrification.

Un dispositif de détection ODM peut être inductif et détecte alors uniquement les particules métalliques. Un dispositif de détection ODM peut être optique ou acoustique et détecte toutes les particules, métalliques ou non métalliques, passant à proximité ainsi que les éventuelles bulles d'air présentes dans le liquide de lubrification.

Un dispositif de détection ODM peut aussi utiliser plusieurs technologies et être par exemple d'une part inductif et d'autre optique ou acoustique. Dans ce cas, ce dispositif de détection ODM peut ainsi détecter toutes les particules passant à proximité et identifier les particules métalliques et, par suite, les particules non métalliques.

En outre, un dispositif de détection ODM peut être combiné avec un dispositif de captation permettant de la sorte d'une part de détecter au moins partiellement les particules passant à proximité du dispositif de détection ODM et d'autre part de capter également au moins partiellement les particules passant à proximité du dispositif de détection *ODM,* les particules captées étant métalliques ou de tout type selon la technologie de captation utilisée.

On connait les documents US 2009/0240471, US 2016/0266006, EP 2014877 et EP 2574905 qui décrivent l'utilisation d'un tel dispositif de détection de particules. Le document US 2009/0240471 décrit un système de détection d'un défaut d'un système mécanique, par exemple une turbine à gaz. Ce système de détection comporte plusieurs capteurs, notamment un dispositif analysant les vibrations et un dispositif de détection, sans captation, de la quantité et de la taille des particules en suspension dans le liquide de lubrification. Le document US 2016/0266006 décrit un système de surveillance d'une boîte de vitesse d'un véhicule. Ce système de surveillance utilise un dispositif de détection, sans captation, de la quantité et de la taille des particules en suspension dans le liquide de lubrification de cette boîte de vitesse. Ce dispositif de détection peut être par exemple optique ou bien magnétique.

Le document EP 2014877 décrit quant à lui un système de surveillance d'une turbine à gaz utilisant plusieurs dispositifs de captation de particules métalliques et non métalliques tels qu'un filtre ou un bouchon magnétique, ainsi qu'au moins un dispositif de détection de particules métalliques et non métalliques circulant avec un liquide de lubrification.

Enfin, le document EP 2574905 décrit un système de surveillance des particules susceptibles de polluer un circuit de lubrification ou bien un système hydraulique. Ce système de surveillance comporte un dispositif optique de détection de particules permettant de mesurer la taille et les quantités de particules circulant avec le circuit de lubrification ou bien dans le système hydraulique.

On connait aussi le document US 2013/0332045 qui décrit un système et un procédé de détection des débris dans un moteur. Selon ce procédé, lors d'un premier niveau de surveillance, un capteur de débris, notamment magnétique, permet de détecter et de caractériser des débris circulant dans un circuit de lubrification. Les dimensions des débris peuvent notamment être estimées et comparées à des dimensions prédéterminées. La masse cumulée des débris de dimensions inférieures aux dimensions prédéterminées peut aussi être estimée et comparée à une masse prédéterminée.

L'arrière plan technologique de l'invention comporte les documents US 2019/0162687, US 2017/0248572, US 2002/0148788, US 6582661 et FR 2508168.

La présente invention a alors pour objet de proposer une méthode et un système de surveillance d'un système mécanique lubrifié visant à s'affranchir des limitations mentionnées ci-dessus, en anticipant les risques d'apparition de dommages sur le système mécanique tout en limitant les risques de déclencher de fausses alertes. La présente invention vise ainsi à améliorer la détection de dommages sur le système mécanique et d'optimiser en conséquence les opérations de maintenance de ce système mécanique.

Un objet selon la présence invention est un procédé de surveillance d'un système mécanique, tel que défini par la revendication 1. Un autre objet selon la présente invention est un système mécanique, tel que défini par la revendication 12.

Un système mécanique comporte généralement des éléments mobiles, voire tournants, tels que des arbres en rotation et des roulements, ainsi que des éléments de transmission de puissance et de réduction ou d'augmentation de vitesse de rotation. Un système mécanique permet par exemple la transmission d'une puissance mécanique d'une installation motrice du véhicule vers un moyen de propulsion du véhicule.

Il est alors essentiel pour le bon fonctionnement du système mécanique et, le cas échéant d'un véhicule comportant ce système mécanique, de lubrifier et de refroidir les éléments tournants et les éléments de transmission de puissance du système mécanique par un liquide de lubrification, par exemple de l'huile. Dans ce but, le système mécanique comporte un système de lubrification muni de :
- au moins un réservoir contenant un liquide de lubrification,
- au moins un circuit de lubrification dans lequel circule le liquide de lubrification et destiné à une lubrification du système mécanique, ledit au moins un circuit de lubrification comportant des conduites dans lesquelles circule le liquide de lubrification,
- au moins un générateur de débit du liquide de lubrification permettant d'alimenter au moins un circuit de lubrification avec le liquide de lubrification, et
- au moins un dispositif de captation de particules.

Chaque circuit de lubrification achemine alors le liquide de lubrification jusqu'aux éléments à lubrifier du système mécanique et peut comporter des gicleurs afin de pulvériser le liquide de lubrification sur ces éléments. Le réservoir du système de lubrification est par exemple constitué par un carter du système mécanique.

Le procédé de surveillance selon l'invention comporte les étapes suivantes :
- mesure d'au moins une première caractéristique de particules détectées circulant dans au moins une conduite d'un circuit de lubrification,
- comparaison d'au moins une dite première caractéristique avec un premier seuil,
- collecte des particules captées par ledit au moins un dispositif de captation si au moins un premier seuil est dépassé,
- mesure d'au moins une seconde caractéristiques des particules captées,
- comparaison d'au moins une seconde caractéristique des particules captées avec un deuxième seuil, et
- maintenance du système mécanique si au moins un deuxième seuil est dépassé.

Le procédé selon l'invention permet tout d'abord d'identifier un risque potentiel d'apparition d'un dommage sur le système mécanique par la mesure et par la comparaison d'au moins une première caractéristique de particules détectées circulant dans une conduite d'un circuit de lubrification sans arrêt du système mécanique, puis d'adapter avantageusement l'opération de maintenance à éventuellement mettre en place pour confirmer ou infirmer ce risque. De plus, les fausses alertes sont également limitées par l'utilisation du procédé selon l'invention.

De la sorte, un démontage du système mécanique immobilisant ce système mécanique, et le cas échéant le véhicule qui comporte ce système mécanique, est ainsi réalisé uniquement en cas de dommage avéré du système mécanique. Le procédé selon l'invention permet en conséquence d'optimiser les opérations de maintenance du système mécanique tant en termes de coût que de temps d'immobilisation du système mécanique.

La mesure d'au moins une première caractéristique de ces particules est effectuée par au moins un dispositif de détection de particules. Cet au moins un dispositif de détection est de préférence agencé dans le flux du liquide de lubrification d'un circuit de lubrification, typiquement dans une conduite d'au moins un circuit de lubrification. Ce dispositif de détection peut être optique, acoustique ou inductif et est par exemple de type ODM. Par exemple, un dispositif de détection de particules est agencé dans une conduite de chaque circuit de lubrification du système de lubrification.

Ce dispositif de détection de particules peut permettre uniquement la détection de particules, sans captation ni blocage de ces particules.

Ce dispositif de détection de particules peut être combiné avec un dispositif de captation permettant d'une part la détection de particules passant à proximité du dispositif de détection et d'autre part la captation au moins partielle des particules passant à proximité, les particules captées pouvant être métalliques ou de tout type selon la technologie de captation utilisée.

En outre, chaque dispositif de détection permet également de caractériser les particules détectées. Les particules détectées peuvent ainsi être caractérisées par une seule première caractéristique, typiquement le nombre de particules détectées, ou bien par plusieurs premières caractéristiques. Chaque première caractéristique peut être choisie parmi le nombre de particules détectées, le débit de particules détectées circulant dans le circuit de lubrification à proximité du dispositif de détection, la taille de chaque particule détectée ou bien la taille maximale d'une particule parmi les particules détectées et la masse de chaque particule détectée ou bien la masse maximale d'une particule parmi les particules détectées.

La taille d'une particule est par exemple proportionnelle à l'amplitude d'un signal électrique reçu par le dispositif de détection. Ce signal électrique est ensuite traité par un calculateur associé au dispositif de détection ou bien intégré à un système électronique du système mécanique, typiquement le système avionique d un aéronef dans le cas d'un système mécanique embarqué dans l'aéronef, afin de déterminer la taille de chaque particule détectée selon une table de conversion. Cette table de conversion est par exemple établie au cours d'essais d'étalonnages préliminaires du dispositif de détection.

Ensuite, au moins une première caractéristique des particules détectées est comparée avec un premier seuil correspondant par exemple par un calculateur. Ce calculateur peut être par exemple intégré au système mécanique ou bien au véhicule comportant ce système mécanique le cas échéant. Ce calculateur est par exemple intégré à un système avionique d'un aéronef comportant le système mécanique.

Le calculateur peut comporter au moins un processeur et au moins une mémoire, au moins un circuit intégré, au moins un système programmable ou bien au moins un circuit logique, ces exemples ne limitant pas la portée donnée à l'expression « calculateur ». Le calculateur peut être un calculateur dédié à la réalisation du procédé selon l'invention ou être un calculateur partagé ayant de multiples fonctions. La mémoire peut par exemple stocker un ou plusieurs premiers seuils correspondant respectivement à une première caractéristique distincte.

Lorsque que plusieurs premières caractéristiques de particules détectées sont déterminées, chaque première caractéristique est de préférence comparée indépendamment des autres premières caractéristiques à un premier seuil spécifique à cette première caractéristique.

Toutefois, une combinaison d'au moins deux premières caractéristiques de particules détectées peut également être comparée à un premier seuil correspondant à une telle combinaison.

Suite à cette comparaison, une collecte des particules captées par ledit au moins un dispositif de captation est réalisée si au moins un premier seuil est dépassé. Un premier seuil est dépassé dès qu'une première caractéristique est supérieure au premier seuil correspondant. Par exemple, un premier seuil est dépassé dès que le nombre de particules détectées est supérieur à un premier seuil, par exemple égal à 80. Un premier seuil est également dépassé dès que le débit de particules détectées circulant dans le circuit de lubrification est supérieur à un premier seuil, par exemple égal à 10 particules par heures, ou bien dès que la taille maximale des particules détectées est supérieure à un premier seuil, par exemple égal au moins une particule supérieure à 800 micromètres (800 µm). Le nombre de particules détectées est égal au nombre cumulé de particules détectées depuis une initialisation de ce nombre effectuée généralement lors d'une opération conséquente de maintenance du système mécanique.

Si une combinaison d'au moins deux premières caractéristiques de particules détectées est utilisée, la collecte des particules captées est réalisée si une telle combinaison est supérieure au premier seuil correspondant à cette combinaison.

Un dispositif de captation peut être un bouchon magnétique, un filtre, une crépine ou encore un tamis agencé sur le système de lubrification. Un dispositif de captation peut aussi être une zone de rétention des particules située dans le réservoir ou bien dans le flux du liquide de lubrification.

La collecte des particules consiste tout d'abord à une inspection d'un ou de plusieurs dispositifs de captation et en cas de présence de particules captées par au moins un dispositif de captation, ces particules captées sont collectées, à savoir retirées dudit au moins un dispositif de captation, en vue d'une analyse.

En outre, un déclenchement d'une alerte peut être réalisé si au moins un premier seuil est dépassé par une première caractéristique des particules détectées. Ce déclenchement d'une alerte peut être réalisé dès qu'au moins un premier seuil est dépassé. Cependant, Ce déclenchement d'une alerte peut être réalisé si au moins un premier seuil est dépassé et après un arrêt dudit système mécanique.

En effet, un dépassement d'un premier seuil signifie qu'un risque de dommage sur le système mécanique existe et doit être confirmé ou infirmé par une analyse complémentaire de particules captées dans le système de lubrification. Dès lors, aucun dommage n'est encore avéré et le fonctionnement du système mécanique n'est pas en danger. Il n'y a donc pas d'urgence à arrêter le système mécanique et le déclenchement d'une alerte peut être différé jusqu'à un arrêt du système mécanique, notamment dans le cas d'un aéronef en vol. Le système mécanique ou bien le véhicule comportant ce système mécanique comporte un dispositif d'alerte afin de réaliser le déclenchement de l'alerte. Cette alerte peut être sonore ou visuelle par exemple, afin d'informer un opérateur ou un équipage dans le cas d'un système mécanique embarqué dans un aéronef de ce risque.

En outre, le système mécanique peut comporter un dispositif de captation magnétique à signalisation associé à un dispositif d'alerte afin d'alerter l'opérateur, ou l'équipage dès qu'une quantité de particules supérieure à un seuil est captée, cette quantité de particules étant significative d'un risque de présence d'un dommage sur le système mécanique.

Le déclenchement d'une alerte suite à un dépassement d'au moins un premier seuil par une première caractéristique des particules détectées étant effectué avant le déclenchement d'une alerte par le dispositif de captation magnétique à signalisation, le procédé selon l'invention permet avantageusement d'anticiper la détection d'un possible risque de dommage et d'éventuellement agir par une inspection ou une opération de maintenance du système mécanique avant qu'un dommage du système mécanique soit avéré ou bien lorsque qu'un tel dommage n'est pas encore trop important.

Suite à la collecte des particules captées par au moins un dispositif de captation du système de lubrification du système mécanique, une analyse de ces particules est réalisée. Tout d'abord, une mesure d'au moins une seconde caractéristiques des particules captées est effectuée. Cette mesure d'au moins une seconde caractéristiques des particules captées peut être réalisée manuellement, typiquement tout d'abord visuellement et complétée par une analyse en laboratoire. Cette mesure d'au moins une seconde caractéristiques des particules captées peut aussi être réalisée de façon totalement automatique.

Chaque seconde caractéristique peut être choisie parmi le nombre de particules captées, la taille de chaque particule captée ou bien la taille maximale d'une particule parmi les particules captées, la masse de chaque particule captée ou bien la masse maximale d'une particule parmi les particules captées, le matériau et la morphologie des particules captées.

La morphologie d'une particule permet de caractériser la forme de la particule. En particulier, la morphologie permet de différencier une particule formée en copeau issu de l'usinage d'un élément du système mécanique qui n'est donc significatif d'un dommage de cet élément et une particule en écaille consécutive par exemple à un arrachement de matière d'un élément du système mécanique représentatif d'un dommage.

Ensuite, une comparaison d'au moins une seconde caractéristique des particules captées avec un deuxième seuil est effectuée par exemple par un calculateur dédié. Un ou plusieurs deuxièmes seuils correspondant respectivement des secondes caractéristiques distinctes peuvent être stockés dans la mémoire du calculateur.

Lorsque plusieurs secondes caractéristiques de particules captées sont déterminées, chaque seconde caractéristique est de préférence comparée indépendamment des autres secondes caractéristiques à un deuxième seuil spécifique à cette seconde caractéristique.

Toutefois, une combinaison d'au moins deux secondes caractéristiques de particules captées peut également être comparée à un deuxième seuil correspondant à une telle combinaison. Notamment, le matériau et la morphologie sont des secondes caractéristiques utilisées en combinaison avec le nombre, la taille et/ou la masse.

Suite à cette comparaison et si au moins un deuxième seuil est dépassé par une seconde caractéristique, une opération de maintenance du système mécanique est réalisée. En effet, si un deuxième seuil est dépassé, à savoir qu'une seconde caractéristique est supérieure au deuxième seuil correspondant, les particules captées confirment qu'un risque de dommage d'au moins un élément tournant ou de transmission mécanique du système mécanique existe. Une opération de maintenance est donc nécessaire afin de démonter au moins partiellement le système mécanique, d'identifier le ou les dommages subis et de réparer le système mécanique.

Par exemple, un deuxième seuil est dépassé dès que le nombre de particules captées est supérieur à un deuxième seuil, par exemple égal à 10, ou bien dès que la taille maximale des particules captées est supérieure à un deuxième seuil, par exemple égal à 800 micromètres (800 µm).

Le procédé peut de plus comprendre une ou plusieurs des caractéristiques qui suivent, prises seules ou en combinaison.

Selon un aspect, si chaque seconde caractéristique des particules captées est inférieure au deuxième seuil correspondant et au moins une des secondes caractéristiques est supérieure à un troisième seuil, le troisième seuil étant inférieur au deuxième seuil, le procédé peut comporter les étapes supplémentaires :
- analyse de ladite au moins une première caractéristique des particules détectées pour identifier si le liquide de lubrification est pollué,
- nettoyage du système mécanique si une pollution du liquide de lubrification est identifiée,
- planification de collectes régulières des particules captées par ledit au moins un dispositif de captation si aucune pollution du liquide de lubrification n'est identifiée.

En effet, la présence des particules dans un ou plusieurs circuits de lubrification ou bien dans le réservoir du système de lubrification peut être due à une pollution du liquide de lubrification et non à un dommage d'un élément du système mécanique. Dès lors, le procédé selon l'invention permet, lors de cette analyse, d'identifier si à partir de la mesures de ladite au moins une première caractéristique des particules détectées si le liquide de lubrification est pollué, évitant ainsi avantageusement d'engager une opération importante de maintenance inutile suite à une telle pollution du liquide de lubrification. Un nettoyage du système mécanique et de chaque circuit de lubrification peut alors être réalisé afin de supprimer cette pollution du liquide de lubrification. Au contraire, si aucune pollution significative du liquide de lubrification n'est identifiée, un risque de dommage sur au moins un élément tournant ou de transmission mécanique du système mécanique reste possible, même s'il n'est pas avéré pour l'instant. En conséquence, une surveillance renforcée du système mécanique est engagée afin de suivre de près ce risque de dommage. Cette surveillance renforcée comporte notamment une planification de collectes régulières et d'analyses des particules captées par au moins un dispositif de captation du système de lubrification du système mécanique.

Cette planification de collectes régulières des particules captées est effectuées avec des intervalles d'inspection réduits par rapport à une procédure de maintenance préventive habituelle afin d'anticiper la détection d'une dégradation du système mécanique.

Lors de l'étape d'analyse de ladite au moins une première caractéristique, une évolution dans le temps d'une première caractéristique des particules détectées est analysée. Ainsi, la présence d'une pollution du liquide de lubrification est identifiée si cette évolution dans le temps d'une première caractéristique des particules détectées comporte une première augmentation dans un premier intervalle de temps, puis un palier sensiblement constant dans un second intervalle de temps. La présence d'une pollution du liquide de lubrification est également détectée si cette évolution dans le temps d'une première caractéristique des particules détectées comporte plusieurs augmentations et plusieurs paliers sensiblement constants successifs formant ainsi une courbe en « marche d'escalier ».

La présence d'une pollution du liquide de lubrification est aussi identifiée si cette évolution dans le temps d'une première caractéristique des particules métalliques détectées comporte une première augmentation dans un premier intervalle de temps, puis une seconde augmentation inférieure à la première augmentation dans un second intervalle de temps, le second intervalle de temps faisant immédiatement suite au premier intervalle de temps. De la sorte, cette évolution dans le temps d'une première caractéristique des particules métalliques détectées est une courbe croissante avec au moins un changement de pente, ladite pente diminuant lorsque le temps augmente. La première caractéristique des particules métalliques détectées évolue par exemple dans le temps selon une courbe logarithmique.

Par contre, aucune pollution du liquide de lubrification n'est identifiée si cette évolution dans le temps d'une première caractéristique des particules détectées est constamment croissante et de façon linéaire.

De même, aucune pollution du liquide de lubrification n'est identifiée si cette évolution dans le temps d'une première caractéristique des particules détectées comporte une première augmentation dans un premier intervalle de temps, puis une seconde augmentation supérieure à la première augmentation dans un second intervalle de temps. De la sorte, cette évolution dans le temps d'une première caractéristique des particules métalliques détectées est une courbe croissante avec au moins un changement de pente, ladite pente augmentant lorsque le temps augmente.

La première caractéristique des particules détectées évolue par exemple dans le temps selon une courbe exponentielle ou bien une courbe parabolique.

Dans ces deux cas, un risque de présence d'un dommage ou d'une dégradation d'au moins un élément du système mécanique reste possible et la surveillance renforcée du système mécanique doit être engagée.

En outre, l'étape d'analyse de ladite au moins une première caractéristique peut être réalisée indépendamment des étapes de collecte des particules captées, de mesure et de comparaison d'au moins une seconde caractéristique, après l'étape de comparaison d'au moins une première caractéristique des particules avec un premier seuil et si au moins un premier seuil est dépassé.

De la sorte, l'étape d'analyse de ladite au moins une première caractéristique permet avantageusement d'identifier l'origine des particules détectées et de discriminer des particules issues d'un dommage d'un élément mobile du système mécanique ou bien d'une pollution du liquide de lubrification, sans intervenir sur le système mécanique et sans arrêt ni démontage de ce système mécanique. Cette étape de détection peut permettre ainsi d'adapter l'opération de maintenance à réaliser en fonction de l'origine de ces particules et également de limiter le risque de fausse alerte, sans que les étapes de collecte des particules captées, de mesure et de comparaison d'au moins une seconde caractéristique soient réalisées.

Cette étape d'analyse de ladite au moins une première caractéristique peut être faite par le calculateur que comporte le système mécanique ou bien le véhicule comportant le système mécanique, éventuellement pendant le fonctionnement du système mécanique. De la sorte, dès l'arrêt du système mécanique, un éventuel nettoyage du système mécanique peut être engagé rapidement si une pollution du liquide de lubrification est identifiée.

Lorsque le système mécanique équipe un aéronef, le procédé selon l'invention peut également comporter une étape de transmission de ladite au moins une première caractéristique des particules détectées au cours d'un vol vers une base au sol munie d'un calculateur afin de réaliser l'étape d'analyse de ladite au moins une première caractéristique. Dans ce cas, la détection d'une éventuelle pollution du liquide de lubrification peut être réalisée avant la fin du vol de l'aéronef et un éventuel nettoyage du système mécanique peut être engagé rapidement si une pollution du liquide de lubrification est identifiée.

L'étape de transmission de ladite au moins une première caractéristique des particules détectées vers une base au sol peut également être réalisée lorsque l'aéronef est au sol.

Selon un autre aspect de l'invention, l'étape de mesure d'au moins une première caractéristique de particules détectées circulant dans au moins une conduite d'un circuit de lubrification peut être limitée aux particules métalliques détectées.

En effet, lorsque les éléments tournants et les éléments de transmission de puissance du système mécanique sont tous métalliques, les particules non métalliques éventuellement détectées ne sont pas significatives d'un dommage de ces éléments et peuvent alors entraîner une détection d'un risque de dommage inexistant. La mesure d'au moins une première caractéristique uniquement des particules métalliques détectées permet de pallier ce risque.

Dans ce cas, le dispositif de détection de particules peut détecter uniquement les particules métalliques ou bien identifier les particules métalliques parmi les particules détectées. Le dispositif de détection peut par exemple être inductif et détecte uniquement les particules métalliques. Le dispositif de détection peut aussi utiliser plusieurs technologies et être par exemple d'une part inductif et d'autre optique ou acoustique afin de détecter toutes les particules passant à proximité et d'identifier les particules métalliques parmi ces particules.

En conséquence, l'étape de comparaison d'au moins une dite première caractéristique avec un premier seuil se limite également à chaque première caractéristique des particules métalliques détectées afin d'identifier un risque de dommages subis par un élément métallique du système mécanique.

De plus, l'étape d'analyse de ladite au moins une première caractéristique des particules détectées afin d'identifier si le liquide de lubrification est pollué est également effectuée uniquement sur les particules métalliques captées.

A contrario, lorsque le système mécanique comporte des éléments métalliques et des éléments non métallique, en céramique par exemple, il est préférable de détecter toutes les particules, métalliques et non métalliques, circulant avec le liquide de lubrification et de comparer les première caractéristiques de ces particules métalliques et non métalliques détectées avec un premier seuil.

Selon un autre aspect de l'invention, l'étape de collecte des particules captées par ledit au moins un dispositif de captation si au moins un premier seuil est dépassé peut également être limitée aux particules métalliques pour les même raisons que précédemment, à savoir lorsque les éléments tournants et les éléments de transmission de puissance du système mécanique sont métalliques.

Un dispositif de captation peut être un bouchon magnétique permettant de capter uniquement des particules métalliques, faciles à collecter une fois le bouchon magnétique retiré.

Un dispositif de captation peut être un filtre, une crépine, un tamis ou une zone de rétention, permettant de capter des particules de tout type, à savoir des particules métalliques et des particules non métalliques. Un tri parmi les particules collectées est alors effectué afin d'identifier et d'isoler les particules métalliques captées. Ce tri peut par exemple être effectué par l'intermédiaire d'un dispositif magnétique.

En conséquence, l'étape de mesure d'au moins une seconde caractéristiques des particules captées et l'étape de comparaison d'au moins une seconde caractéristique des particules captées avec un deuxième seuil se limitent également aux particules métalliques captées afin de confirmer ou non le risque de dommages subis par un élément métallique du système mécanique.

A contrario, lorsque le système mécanique comporte des éléments métalliques et des éléments non métallique, en céramique par exemple, il est préférable de collecter toutes les particules, métalliques et non métalliques, captées par chaque dispositif de captation et de comparer les secondes caractéristiques de ces particules métalliques et non métalliques captées avec un deuxième seuil.

La présente invention vise également un système de surveillance pour surveiller un système mécanique lubrifié, le système de surveillance comportant un système de lubrification du système mécanique, le système de lubrification étant muni de :
- au moins un réservoir contenant un liquide de lubrification,
- au moins un circuit de lubrification, dans lequel circule le liquide de lubrification et destiné à une lubrification du système mécanique, ledit au moins un circuit de lubrification, comportant des gicleurs, des conduites dans lesquelles circule le liquide de lubrification,
- au moins un générateur de débit du liquide de lubrification permettant d'alimenter au moins un circuit de lubrification, avec le liquide de lubrification, et
- au moins un dispositif de captation de particules.

Le système de surveillance comporte également au moins un dispositif de détection de particules agencé dans au moins un circuit de lubrification, ainsi qu'au moins un calculateur et un dispositif d'alerte, le système de surveillance étant configuré pour mettre en œuvre le procédé de surveillance d'un système mécanique lubrifié précédemment décrit.

Un dispositif de captation de particules peut comporter au moins un bouchon magnétique ou au moins un bouchon magnétique à signalisation ou encore un filtre, une crépine, un tamis ou une zone de rétention.

Le système de lubrification peut aussi comporter au moins un dispositif susceptible de piéger des particules métalliques non métalliques, par exemple un dispositif de filtration, tel un filtre, une crépine et un tamis, ou bien dispositif d'échange thermique. Le dispositif de détection est agencé de préférence en aval du générateur de débit et en amont d'un tel dispositif susceptible de piéger ces particules afin de limiter le risque que des particules soient piégées par un tel dispositif.

Un dispositif de détection de particules est par exemple un dispositif inductif, acoustique ou bien optique. Un dispositif de détection de particules peut être un dispositif de type ODM.

Le procédé de surveillance et le système de surveillance selon l'invention peuvent aussi être appliqués à un système mécanique lubrifié par barbotage, au moins un dispositif de détection étant par exemple agencé dans un lieu de circulation du liquide de lubrification afin de détecter les premières caractéristiques des particules circulant avec le liquide de lubrification.

La présente invention vise aussi un système mécanique comportant :
- des éléments tournants,
- des éléments de transmission de puissance et de réduction ou d'augmentation de vitesse de rotation, et
- un système de surveillance pour surveiller un système mécanique lubrifié tel que précédemment décrit.

Le système mécanique peut être par exemple une boîte de transmission principale de puissance d'un aéronef à voilure tournante.

La présente invention vise enfin un aéronef comportant un tel système mécanique,

L'invention et ses avantages apparaîtront avec plus de détails dans le cadre de la description qui suit avec des exemples donnés à titre illustratif en référence aux figures annexées qui représentent :
- les figures 1 à 2, des exemples de systèmes mécaniques, et
- les figures 3 à 7, des courbes d'évolutions d'une première caractéristique de particules détectées.

Les éléments présents dans plusieurs figures distinctes sont affectés d'une seule et même référence.

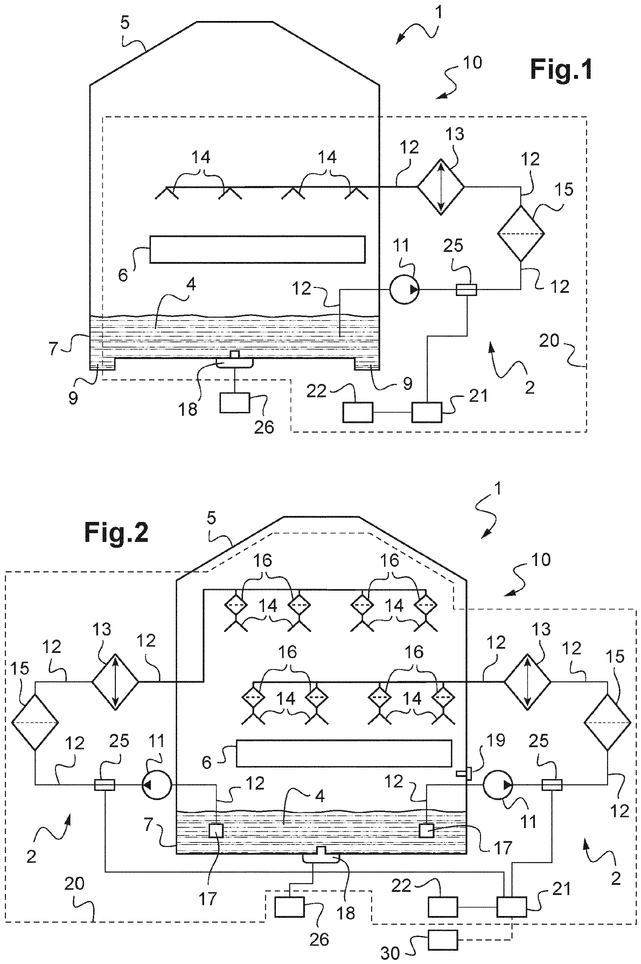

Les figures 1 et 2 représentent un système mécanique 1 comportant un système de surveillance 20 pour surveiller ce système mécanique 1. Ce système mécanique 1 comporte notamment un carter 5 et des éléments mécaniques 6 mobiles, en particulier des éléments tournants, tels que des arbres et des roulements, ainsi que des éléments de transmission de puissance et de réduction ou d'augmentation de vitesse de rotation, tels que des pignons et/ou des engrenages. Ce système mécanique 1 est par exemple une boîte de transmission principale de puissance équipant un aéronef à voilure tournante.

Le système de surveillance 20 comporte un système de lubrification 10, au moins un dispositif de détection 25 de particules agencé dans le système de lubrification 10, un calculateur 21 et un dispositif d'alerte 22.

Selon le premier exemple de système mécanique 1 représenté sur la figure 1, le système de lubrification 10 comporte un seul circuit de lubrification 2 alors que le second exemple de système mécanique 1 représenté sur la figure 2 comporte deux circuits de lubrification 2 identiques. Chaque circuit de lubrification 2 permet de lubrifier et de refroidir simultanément les éléments mécaniques 6 mobiles du système mécanique 1.

De façon commune, ces deux exemples, le système de lubrification 10 comporte un réservoir 7 formé par une partie inférieur du carter 5 du système mécanique 1 et contenant un liquide de lubrification 4, tel que de l'huile ainsi qu'un bouchon magnétique 18 agencé au fond de ce réservoir 7. Chaque circuit de lubrification 2 du système de lubrification 10 comporte également un générateur de débit 11, telle une pompe, un dispositif de refroidissement 13 formé par un échangeur thermique, des gicleurs 14, au moins un dispositif de captation 15-19 de particules et des conduites 12 reliant ces différents composants.

Un dispositif de détection 25 de particules du système de surveillance 20 est également agencé sur chaque circuit de lubrification 2 sur une conduite 12 positionnée en aval du générateur de débit 11 et en amont d'un dispositif de captation 15.

Le générateur de débit 11 permet d'aspirer le liquide de lubrification 4 situé dans le réservoir 7 et d'alimenter avec ce liquide de lubrification 4 chaque circuit de lubrification 2 jusqu'à sa pulvérisation sur les éléments mécaniques 6 mobiles du système mécanique 1 par l'intermédiaire des gicleurs 14. Le dispositif de refroidissement 13 permet de refroidir le liquide de lubrification 4 avant qu'il atteigne les gicleurs 14. Le dispositif de refroidissement 13 est aussi susceptible de piéger temporairement, voire définitivement, des particules métalliques ou non métalliques circulant dans ce dispositif de refroidissement 13. Certaines de ces particules peuvent être réintroduites ultérieurement et de façon aléatoire dans le circuit de lubrification 2.

Chaque dispositif de captation 15-19 est agencé sur un circuit de lubrification 2 afin de capter certaines particules circulant avec le liquide de lubrification 4 de sorte à limiter leur circulation dans chaque circuit de lubrification 2. Un dispositif de captation 15-17 peut être un dispositif de captation mécanique munis d'orifices et permettant de bloquer certaines particules métalliques ou non métalliques ayant des dimensions supérieures ou égales à celles de ces orifices. Un dispositif de captation 18,19 peut également être magnétique et être agencé dans le système de lubrification 4 afin de capter et de retenir uniquement les particules métalliques passant à proximité.

Selon l'exemple de la figure 1, l'unique circuit de lubrification 2 comporte un dispositif de captation mécanique 15 formé par un dispositif de filtration, par exemple un filtre, agencé en aval du dispositif de détection 25 et en amont du dispositif de refroidissement 13, ainsi qu'un dispositif de captation magnétique 18 formé par un bouchon magnétique à signalisation agencé dans le fond du réservoir 7. Ce dispositif de captation magnétique 18 est relié à un dispositif d'alerte 26 secondaire qui peut déclencher une alerte dès qu'une quantité prédéterminée de particules métalliques a été captée par le dispositif de captation magnétique 18.

Un dispositif de captation supplémentaire 9 est agencé dans le réservoir 7. Ce dispositif de captation supplémentaire 9 constitue une zone de rétention de particules et comporte deux cavités agencées au fond du réservoir 7 et permettant de piéger des particules métalliques ou non métalliques par gravité. Un bouchon (non représenté sur la figure 1) peut être agencé dans chaque cavité afin de collecter les particules accumulées.

Selon l'exemple de la figure 2, chaque circuit de lubrification 2 comporte plusieurs dispositifs de captation 15-17 mécanique et deux dispositifs de captation magnétique 18-19. Un premier dispositif de captation 17 mécanique est par exemple une crépine agencée dans le réservoir 7, à l'extrémité d'une conduite 12, à savoir en amont du générateur de débit 11, bloquant certaines particules et évitant qu'elles entrent dans le circuit de lubrification 2. Un deuxième dispositif de captation 15 mécanique est par exemple un filtre agencé en aval du dispositif de détection 25 et en amont du dispositif de refroidissement 13. Des troisièmes dispositifs de captation 15 mécanique sont par exemple des crépines agencées en aval du dispositif de refroidissement 13 et en amont respectivement des gicleurs 14. Chaque troisième dispositif de captation 15 protège de la sorte un gicleur 14 en limitant le risque qu'une ou plusieurs particules viennent obstruer au moins partiellement ce gicleur 14.

Les deux dispositifs de captation magnétique 18-19 comportent un bouchon magnétique à signalisation 18, identique à celui de l'exemple de la figure 1, agencé dans le fond du réservoir 7 et relié à un dispositif d'alerte 26 secondaire ainsi qu'un bouchon magnétique 19 agencé dans une paroi du carter 5, à proximité d'éléments mécaniques 6 mobiles. Chaque bouchon magnétique 18,19 permet de capter les particules métalliques se trouvant ou se déplaçant à proximité de lui.

Le système de surveillance 20 permet de surveiller le système mécanique 1 grâce à chaque dispositif de détection 25 tout d'abord en détectant des particules circulant dans chaque circuit de lubrification 2 et passant à proximité de ce dispositif de détection 25 et en mesurant simultanément au moins une première caractéristique de ces particules détectées. Ensuite, une analyse de ladite au moins une première caractéristique des particules détectées peut être réalisée afin d'identifier éventuellement un risque d'apparition d'une dégradation d'au moins un élément mécanique 6 mobile du système mécanique 1.

Dans ce but, le système de surveillance 20 peut mettre en œuvre un procédé de surveillance 20 du système mécanique 1 comportant les étapes suivantes.

Tout d'abord, une étape de mesure d'au moins une première caractéristique de particules détectées par le dispositif de détection 25 est réalisée. Le dispositif de détection 25 peut être optique ou acoustique afin de détecter et de mesurer les particules métalliques ou non métalliques circulant avec le liquide de lubrification 4 et passant à proximité du dispositif de détection 25. Le dispositif de détection 25 peut également être inductif afin de détecter et de mesurer uniquement les particules métalliques circulant avec le liquide de lubrification 4 et passant à proximité du dispositif de détection 25. Chaque première caractéristique des particules détectées est représentatif d'une quantité cumulée de particules passant à proximité du dispositif de détection 25.

Ensuite, une étape de comparaison d'au moins une première caractéristique des particules détectées par le dispositif de détection 25 avec un premier seuil est réalisée par l'intermédiaire du calculateur 21. Une première caractéristique des particules détectées est par exemple le nombre des particules détectées, le débit des particules, la taille maximale parmi les tailles des particules détectées. Chaque première caractéristique des particules détectées est représentative d'une quantité cumulée de particules captées par un ou plusieurs dispositifs de captation 15-19. Un premier seuil correspondant à chaque première caractéristique est par exemple stocké dans une mémoire du calculateur 21.

Suite à cette étape de comparaison et si au moins un premier seuil est dépassé, une étape de collecte de particules captées par au moins un dispositif de captation 15-19, voire par chaque dispositif de captation 15-19 d'un circuit de lubrification 2, est réalisée. Chaque premier seuil est défini pour détecter un tel risque avant le déclenchement d'une alerte par l'intermédiaire du dispositif d'alerte 26 secondaire relié au bouchon magnétique à signalisation 18.

La collecte des particules consiste, après une inspection d'un ou de plusieurs dispositifs de captation 15-19, de collecter les particules captées par l'intermédiaire d'au moins un dispositif de captation 15-19. De la sorte, les particules captées sont retirées dudit au moins un dispositif de captation 15-19 en vue d'une analyse. Si seules les particules métalliques captées doivent être analysées, un tri entre les particules métalliques et les particules non métalliques captées par les dispositifs de captation 15-17 est nécessaire, ce tri étant par exemple réalisé par l'intermédiaire d'un dispositif magnétique.

Ensuite, une étape de mesure de ladite au moins une seconde caractéristique des particules captées est réalisée par un dispositif adapté. Une seconde caractéristique peut être le nombre et/ou la taille des particules captées. Concernant la taille des particules captées, l'étape de mesure peut être réalisée manuellement, par exemple par l'intermédiaire d'une loupe de précision graduée ou bien de façon automatique. Chaque seconde caractéristique des particules captées est significative d'une quantité cumulée de particules captées par un ou plusieurs dispositifs de captation 15-19.

Une étape de comparaison d'au moins une seconde caractéristique des particules captées avec un deuxième seuil peut alors être réalisée par l'intermédiaire d'un calculateur dédié. Un deuxième seuil correspondant à chaque seconde caractéristique est par exemple stocké dans une mémoire du calculateur 21.

En fonction de cette comparaison un risque de dégradation d'un élément mécanique 6 du système mécanique 1 peut être identifié et une opération de maintenance du système mécanique 1 éventuellement réalisée pour confirmer ou infirmer la présence de ce risque de dégradation. En l'occurrence dès qu'une seconde caractéristique est supérieure à un deuxième seuil, une étape de maintenance système mécanique 1 est réalisée.

La valeur d'un deuxième seuil peut être déterminée par essai ou bien par retour d'expérience. On sait en effet que de particules captées de dimensions importantes sont généralement significatives de la présence d'une dégradation du système mécanique de même qu'un nombre important de particules captées.

En outre, une alerte peut aussi être déclenchée par l'intermédiaire du dispositif d'alerte 22 afin d'avertir un opérateur qu'un premier seuil est dépassé et qu'une collecte de particules captées par un ou plusieurs dispositifs de captation 15-19 est nécessaire. Cette alerte peut être déclenchée dès qu'un premier seuil est dépassé ou bien après un arrêt du système mécanique suivant un tel dépassement du premier seuil.

Par ailleurs, la comparaison d'au moins une première caractéristique avec un premier seuil a permis d'identifier un risque de dégradation d'un élément mécanique 6 du système mécanique 1 et la comparaison d'au moins une seconde caractéristique avec un deuxième seuil peut permettre d'évaluer si ce risque est avéré ou non. Cependant, la mesure de ladite au moins une première caractéristique peut être un premier signe qu'une telle dégradation d'un élément mécanique 6 du système mécanique 1 se prépare ou bien être consécutif à une pollution du liquide de lubrification. Dès lors, une étape d'analyse de ladite au moins une première caractéristique des particules détectées peut permettre d'identifier si une pollution du liquide de lubrification 4 est éventuellement à l'origine de la mesure de la première caractéristique supérieure au premier seuil.

Une telle étape d'analyse peut être réalisée directement par le calculateur 21 du système de surveillance 20. Une telle étape d'analyse peut également être réalisée en dehors du système de surveillance 20, par exemple par un dispositif déporté 30. Dans ce cas, la mesure de ladite au moins une première caractéristique des particules détectées doit être transférée à ce dispositif déporté 30, par exemple par une liaison sans fil. Le procédé de surveillance 20 du système mécanique 1 peut alors comporter une étape de transmission de ladite au moins une première caractéristique des particules détectées vers le dispositif déporté 30.

Lorsque le système mécanique 1 équipe un aéronef, le dispositif déporté 30 est par exemple une base au sol, permettant ainsi de ne pas mobiliser le calculateur 21 du système de surveillance 20 pour cette analyse, en particulier si ce calculateur 21 est partagé avec d'autres dispositifs de l'aéronef.

Lorsque le système mécanique 1 équipe un aéronef, le dispositif déporté 30 peut également permettre uniquement de visualiser des courbes issues de l'étape d'analyse, l'aéronef étant par exemple encore en vol ou bien posé au sol. L'analyse est alors réalisée par un calculateur 21 embarqué dans l'aéronef, par exemple un calculateur 21 intégré au système avionique de l'aéronef.

Cette étape d'analyse peut être réalisée par exemple, suite à la comparaison d'au moins une seconde caractéristique des particules captées avec un deuxième seuil si à la fois chaque seconde caractéristique est inférieure au deuxième seuil et au moins une seconde caractéristique est supérieure à un troisième seuil. Le troisième seuil est inférieur au deuxième seuil.

Cette étape d'analyse peut aussi être réalisée après l'étape de comparaison d'au moins une premier caractéristique des particules détectées avec un premier seuil et dès qu'un premier seuil est dépassé.

Dans tous les cas, lors de cette étape d'analyse, une pollution du liquide de lubrification 4 peut être identifiée à partir de la mesure d'au moins une première caractéristique.

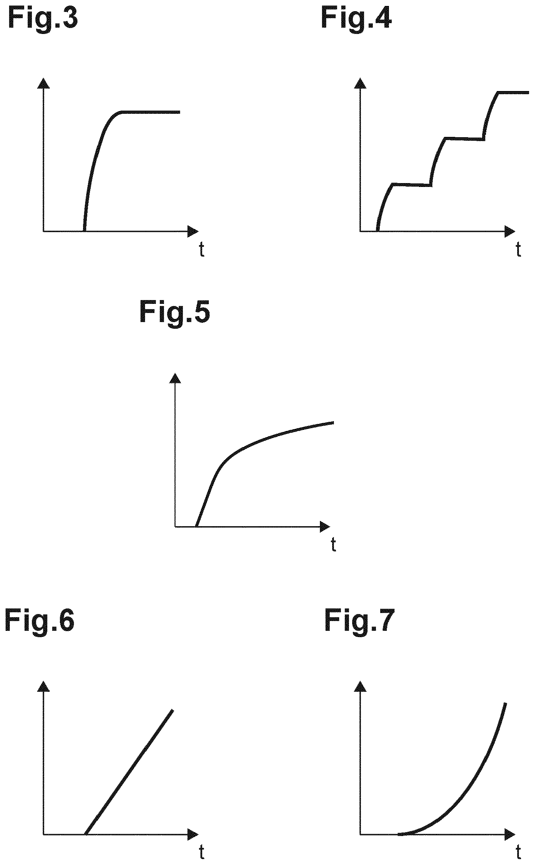

La présence d'une pollution du liquide de lubrification peut être identifiée si une évolution dans le temps d'une première caractéristique des particules détectées comporte une première augmentation dans un premier intervalle de temps, puis un palier sensiblement constant dans un second intervalle de temps, comme représenté sur la figure 3, le second intervalle de temps faisant suite au premier intervalle de temps.

Une évolution dans le temps d'une première caractéristique des particules détectées peut également être représentative d'une pollution si plusieurs augmentations et plusieurs paliers sensiblement constant se succèdent, comme représenté sur la figure 4. Cette évolution prend ainsi la forme caractéristique de marches d'escalier.

La présence d'une pollution du liquide de lubrification peut aussi être identifiée si une évolution dans le temps d'une première caractéristique des particules métalliques détectées comporte une première augmentation dans un premier intervalle de temps, puis une seconde augmentation inférieure à ladite première augmentation dans un second intervalle de temps, comme représenté sur la figure 5, le second intervalle de temps faisant suite au premier intervalle de temps.

A contrario, aucune pollution du liquide de lubrification n'est identifiée lorsqu'une évolution dans le temps d'une première caractéristique des particules détectées est constamment croissante de façon linéaire, comme représenté sur la figure 5.

De même, aucune pollution du liquide de lubrification n'est identifiée lorsqu'une évolution dans le temps d'une première caractéristique des particules détectées comporte une première augmentation dans un premier intervalle de temps, puis une seconde augmentation supérieure à la première augmentation dans un second intervalle de temps, comme représenté sur la figure 6. Une telle évolution dans le temps d'une première caractéristique a par exemple la forme d'une courbe parabolique ou d'une courbe exponentielle.

Suite à cette étape d'analyse, une étape de nettoyage du système mécanique 1 peut être réalisée si une pollution du liquide de lubrification 4 est identifiée, afin notamment d'éliminer cette pollution.

Dans le cas contraire, si aucune pollution du liquide de lubrification n'est identifiée, un risque de présence d'un dommage ou d'une dégradation d'au moins un élément du système mécanique existe et une étape de planification de collectes régulières des particules captées par au moins un dispositif de captation 15-19 peut être mise en œuvre. De la sorte, une surveillance renforcée du système mécanique est assurée afin de suivre la présence ou l'apparition possible d'une dégradation d'un élément mécanique 6. Naturellement, la présente invention est sujette à de nombreuses variations quant à sa mise en œuvre. Bien que plusieurs modes de réalisation aient été décrits, on comprend bien qu'il n'est pas concevable d'identifier de manière exhaustive tous les modes possibles. Il est bien sûr envisageable de remplacer un moyen décrit par un moyen équivalent sans sortir du cadre défini par les revendications.

## Revendications

1. Procédé de surveillance d'un système mécanique (1), ledit système mécanique (1) comportant un système de lubrification (10) muni de :
- au moins un réservoir (7) contenant un liquide de lubrification (4),
- au moins un circuit de lubrification (2) dans lequel circule ledit liquide de lubrification (4) et destiné à une lubrification dudit système mécanique (1), ledit au moins un circuit de lubrification (2) comportant des conduites (12) dans lesquelles circule ledit liquide de lubrification (4),
- au moins un générateur de débit (11) dudit liquide de lubrification (4) permettant d'alimenter au moins un circuit de lubrification (2) avec ledit liquide de lubrification (4), et
- au moins un dispositif de captation (15-19) de particules,
**caractérisé en ce que** ledit procédé comporte les étapes suivantes :
- mesure d'au moins une première caractéristique de particules détectées circulant dans au moins une conduite (12) d'un circuit de lubrification (2),
- comparaison d'au moins une première caractéristique desdites particules détectées avec un premier seuil,
- collecte de particules captées par ledit au moins un dispositif de captation (15-19) si au moins un premier seuil est dépassé,
- mesure d'au moins une seconde caractéristique desdites particules captées,
- comparaison d'au moins une seconde caractéristique desdites particules captées avec un deuxième seuil, et
- maintenance dudit système mécanique (1) si au moins un deuxième seuil est dépassé,
et, si ladite au moins une seconde caractéristique desdites particules captées est inférieure audit deuxième seuil et au moins une seconde caractéristique parmi ladite au moins une seconde caractéristique desdites particules captées est supérieure à un troisième seuil, ledit troisième seuil étant inférieur audit deuxième seuil, ledit procédé comporte des étapes supplémentaires :
- analyse de ladite au moins une première caractéristique desdites particules détectées pour identifier si ledit liquide de lubrification est pollué,
- nettoyage dudit système mécanique (1) si une pollution dudit liquide de lubrification est identifiée, et
- planification de collectes régulières desdites particules captées par ledit au moins un dispositif de captation (15-19) si aucune pollution dudit liquide de lubrification n'est identifiée.

2. Procédé selon la revendication 1,
**caractérisé en ce qu'**un déclenchement d'une alerte est réalisé après un arrêt dudit système mécanique (1) si au moins un premier seuil est dépassé.

3. Procédé selon l'une quelconque des revendications 1 à 2, **caractérisé en ce que** lors de ladite étape d'analyse de ladite au moins une première caractéristique, une présence d'une pollution dudit liquide de lubrification est identifiée si une évolution dans le temps d'une première caractéristique desdites particules détectées est une courbe croissante avec au moins un changement de pente, ladite pente diminuant lorsque le temps augmente.

4. Procédé selon l'une quelconque des revendications 1 à 3, **caractérisé en ce que** lors de ladite étape d'analyse de ladite au moins une première caractéristique, une présence d'une pollution dudit liquide de lubrification est identifiée si une évolution dans le temps d'une première caractéristique desdites particules détectées comporte une première augmentation dans un premier intervalle de temps, puis un palier dans un second intervalle de temps ou bien si ladite évolution comporte plusieurs augmentations et plusieurs paliers successifs.

5. Procédé selon l'une quelconque des revendications 1 à 3, **caractérisé en ce que** lors de ladite étape d'analyse de ladite au moins une première caractéristique, aucune pollution dudit liquide de lubrification n'est identifiée si une évolution dans le temps d'une première caractéristique desdites particules détectées est constamment croissante de façon linéaire.

6. Procédé selon l'une quelconque des revendications 1 à 3, **caractérisé en ce que** lors de ladite étape d'analyse de ladite au moins une première caractéristique, aucune pollution dudit liquide de lubrification n'est identifiée si une évolution dans le temps d'une première caractéristique desdites particules détectées est une courbe croissante avec au moins un changement de pente, ladite pente augmentant lorsque le temps augmente.

7. Procédé selon l'une quelconque des revendications 1 à 6, **caractérisé en ce que** lorsque ledit système mécanique (1) équipe un aéronef, ledit procédé comporte une étape de transmission de ladite au moins une première caractéristique desdites particules détectées vers une base au sol afin de réaliser ladite étape d'analyse de ladite au moins une première caractéristique.

8. Procédé selon l'une quelconque des revendications 1 à 7, **caractérisé en ce qu'**une première caractéristique desdites particules détectées est à choisir parmi le nombre, la taille et le débit desdites particules et qu'une seconde caractéristique desdites particules captées est à choisir parmi le nombre, la taille, la masse, le matériau et la morphologie desdites particules.

9. Procédé selon l'une quelconque des revendications 1 à 8, **caractérisé en ce que** ladite étape de mesure d'au moins une première caractéristique de particules détectées circulant dans au moins une conduite (12) d'un circuit de lubrification (2) se limite aux particules métalliques détectées.

10. Procédé selon l'une quelconque des revendications 1 à 8, **caractérisé en ce que** ladite étape de mesure d'au moins une première caractéristique de particules détectées circulant dans au moins une conduite (12) d'un circuit de lubrification (2) se limite aux particules métalliques détectées et ladite étape d'analyse de ladite au moins une première caractéristique desdites particules détectées pour identifier si ledit liquide de lubrification est pollué se limite aux particules métalliques détectées.

11. Procédé selon l'une quelconque des revendications 1 à 10, **caractérisé en ce que** ladite étape de collecte de particules captées par ledit au moins un dispositif de captation (15-19) si au moins un premier seuil est dépassé se limite aux particules métalliques captées.

12. Système de surveillance (20) pour surveiller un système mécanique (1), ledit système de surveillance (20) comportant un système de lubrification (10) dudit système mécanique (1), ledit système de lubrification (10) étant muni de :
- au moins un réservoir (7) contenant un liquide de lubrification (4),
- au moins un circuit de lubrification (2) dans lequel circule ledit liquide de lubrification (4) et destiné à une lubrification dudit système mécanique (1), ledit au moins un circuit de lubrification (2) comportant des conduites (12) dans lesquelles circule ledit liquide de lubrification (4),
- au moins un générateur de débit (11) dudit liquide de lubrification (4) permettant d'alimenter au moins un circuit de lubrification (2) avec ledit liquide de lubrification (4), et
- au moins un dispositif de captation (15-19) de particules,
**caractérisé en ce que** ledit système de surveillance (20) comporte au moins un dispositif de détection (25) de particules agencé dans au moins un circuit de lubrification (2) ainsi qu'au moins un calculateur (21) et un dispositif d'alerte (22), ledit système de surveillance (20) étant configuré pour mettre en œuvre le procédé selon l'une quelconque des revendications 1 à 11.

13. Système de surveillance (20) selon la revendication 12, **caractérisé en ce que** ledit au moins dispositif de captation (15-19) de particules comporte au moins un bouchon magnétique (19) ou au moins un bouchon magnétique électrique (18).

14. Système de surveillance (20) selon l'une quelconque des revendications 12 à 13,
**caractérisé en ce que** ledit système de lubrification (10) comporte au moins un dispositif (13) susceptible de piéger desdites particules, ledit dispositif de détection (25) étant agencé en amont dudit au moins un dispositif (13) susceptible de piéger desdites particules.

15. Système de surveillance (20) selon l'une quelconque des revendications 12 à 14,
**caractérisé en ce qu'**au moins un dispositif de détection (25) de particules est inductif, acoustique ou bien optique.

16. Système mécanique (1) comportant :
- des éléments tournants (6),
- des éléments (6) de transmission de puissance et de réduction ou d'augmentation de vitesse de rotation, et
- un système de surveillance (20),
**caractérisé en ce que** ledit système de surveillance (20) est selon l'une quelconque des revendications 12 à 15.

17. Système mécanique (1) selon la revendication 16,
**caractérisé en ce que** ledit système mécanique (1) est une boîte de transmission de puissance d'un aéronef.

18. Aéronef comportant un système mécanique (1),
**caractérisé en ce que** ledit système mécanique (1) est selon l'une quelconque des revendications 16 à 17.

## Patentansprüche

1. Verfahren zum Überwachen eines mechanischen Systems (1), wobei das mechanische System (1) ein Schmiersystem (10) umfasst, das versehen ist mit:
- mindestens einem Behälter (7), der eine Schmierflüssigkeit (4) enthält,
- mindestens einem Schmierkreislauf (2), in dem die Schmierflüssigkeit (4) zirkuliert und der für eine Schmierung des mechanischen Systems (1) bestimmt ist, wobei der mindestens eine Schmierkreislauf (2) Leitungen (12) aufweist, in denen die Schmierflüssigkeit (4) zirkuliert,
- mindestens einem Durchflusserzeuger (11) für die Schmierflüssigkeit (4), der es ermöglicht, mindestens einen Schmierkreislauf (2) mit der Schmierflüssigkeit (4) zu versorgen, und
- mindestens einer Vorrichtung (15-19) zum Auffangen von Partikeln,
**dadurch gekennzeichnet, dass** das Verfahren die folgenden Schritte umfasst:
- Messen mindestens eines ersten Merkmals von erfassten Partikeln, die in mindestens einer Leitung (12) eines Schmierkreislaufs (2) zirkulieren,
- Vergleichen von mindestens einem ersten Merkmal der erfassten Partikel mit einem ersten Schwellenwert,
- Sammeln von Partikeln, die von der mindestens einen Auffangvorrichtung (15-19) aufgefangen werden, wenn mindestens ein erster Schwellenwert überschritten ist,
- Messen mindestens eines zweiten Merkmals der aufgefangenen Partikel,
- Vergleichen mindestens eines zweiten Merkmals der aufgefangenen Partikel mit einem zweiten Schwellenwert, und
- Warten des mechanischen Systems (1), wenn mindestens ein zweiter Schwellenwert überschritten ist,
wobei, wenn das mindestens eine zweite Merkmal der erfassten Partikel unter dem zweiten Schwellenwert liegt und mindestens ein zweites Merkmal des mindestens einen zweiten Merkmals der erfassten Partikel über einem dritten Schwellenwert liegt, wobei der dritte Schwellenwert unter dem zweiten Schwellenwert liegt, das Verfahren weitere Schritte umfasst:
- Analysieren des mindestens einen ersten Merkmals der erfassten Partikel, um zu identifizieren, ob die Schmierflüssigkeit verunreinigt ist,
- Reinigen des mechanischen Systems (1), wenn eine Verunreinigung der Schmierflüssigkeit identifiziert wird, und
- Planen regelmäßiger Sammlungen der durch die mindestens eine Auffangvorrichtung (15-19) aufgefangenen Partikel, wenn keine Verunreinigung der Schmierflüssigkeit identifiziert wird.

2. Verfahren nach Anspruch 1,
**dadurch gekennzeichnet, dass** eine Alarmauslösung nach einem Anhalten des mechanischen Systems (1) durchgeführt wird, wenn mindestens ein erster Schwellenwert überschritten ist.

3. Verfahren nach einem der Ansprüche 1 bis 2,
**dadurch gekennzeichnet, dass** bei dem Schritt des Analysierens des mindestens einen ersten Merkmals ein Vorhandensein einer Verunreinigung der Schmierflüssigkeit identifiziert wird, wenn ein zeitlicher Verlauf eines ersten Merkmals der erfassten Partikel eine ansteigende Kurve mit mindestens einer Änderung der Steigung ist, wobei die Steigung mit zunehmender Zeit abnimmt.

4. Verfahren nach einem der Ansprüche 1 bis 3,
**dadurch gekennzeichnet, dass** bei dem Schritt des Analysierens des mindestens einen ersten Merkmals ein Vorhandensein einer Verunreinigung der Schmierflüssigkeit identifiziert wird, wenn ein zeitlicher Verlauf eines ersten Merkmals der erfassten Partikel einen ersten Anstieg in einem ersten Zeitintervall und dann ein Plateau in einem zweiten Zeitintervall aufweist oder wenn der Verlauf mehrere aufeinanderfolgende Anstiege und mehrere Plateaus aufweist.

5. Verfahren nach einem der Ansprüche 1 bis 3,
**dadurch gekennzeichnet, dass** bei dem Schritt des Analysierens des mindestens einen ersten Merkmals keine Verunreinigung der Schmierflüssigkeit identifiziert wird, wenn ein zeitlicher Verlauf eines ersten Merkmals der erfassten Partikel konstant linear ansteigend ist.

6. Verfahren nach einem der Ansprüche 1 bis 3,
**dadurch gekennzeichnet, dass** bei dem Schritt des Analysierens des mindestens einen ersten Merkmals keine Verunreinigung der Schmierflüssigkeit identifiziert wird, wenn ein zeitlicher Verlauf eines ersten Merkmals der erfassten Partikel eine ansteigende Kurve mit mindestens einer Änderung der Steigung ist, wobei die Steigung mit zunehmender Zeit zunimmt.

7. Verfahren nach einem der Ansprüche 1 bis 6,
**dadurch gekennzeichnet, dass**, wenn das mechanische System (1) zu einem Luftfahrzeug gehört, das Verfahren einen Schritt des Übertragens des mindestens einen ersten Merkmals der erfassten Partikel zu einer Basis am Boden umfasst, um den Schritt des Analysierens des mindestens einen ersten Merkmals durchzuführen.

8. Verfahren nach einem der Ansprüche 1 bis 7,
**dadurch gekennzeichnet, dass** ein erstes Merkmal der erfassten Partikel aus der Anzahl, der Größe und dem Durchsatz der Partikel auszuwählen ist und dass ein zweites Merkmal der erfassten Partikel aus der Anzahl, der Größe, der Masse, dem Material und der Morphologie der Partikel auszuwählen ist.

9. Verfahren nach einem der Ansprüche 1 bis 8,
**dadurch gekennzeichnet, dass** der Schritt des Messens mindestens einer ersten Eigenschaft von erfassten Partikeln, die in mindestens einer Leitung (12) eines Schmierkreislaufs (2) zirkulieren, auf die erfassten Metallpartikel beschränkt ist.

10. Verfahren nach einem der Ansprüche 1 bis 8,
**dadurch gekennzeichnet, dass** der Schritt des Messens mindestens einer ersten Eigenschaft von erfassten Partikeln, die in mindestens einer Leitung (12) eines Schmierkreislaufs (2) zirkulieren, auf die erfassten Metallpartikel beschränkt ist und der Schritt des Analysierens der mindestens einen ersten Eigenschaft der erfassten Partikel, um zu identifizieren, ob die Schmierflüssigkeit verunreinigt ist, auf die erfassten Metallpartikel beschränkt ist.

11. Verfahren nach einem der Ansprüche 1 bis 10,
**dadurch gekennzeichnet, dass** der Schritt des Sammelns von Partikeln, die von der mindestens einen Auffangvorrichtung (15-19) aufgefangen werden, wenn mindestens ein erster Schwellenwert überschritten wird, auf die aufgefangenen Metallpartikel beschränkt ist.

12. Überwachungssystem (20) zum Überwachen eines mechanischen Systems (1), wobei das Überwachungssystem (20) ein Schmiersystem (10) für das mechanische System (1) umfasst, wobei das Schmiersystem (10) versehen ist mit:
- mindestens einem Behälter (7), der eine Schmierflüssigkeit (4) enthält,
- mindestens einem Schmierkreislauf (2), in dem die Schmierflüssigkeit (4) zirkuliert und der für eine Schmierung des mechanischen Systems (1) bestimmt ist, wobei der mindestens eine Schmierkreislauf (2) Leitungen (12) aufweist, in denen die Schmierflüssigkeit (4) zirkuliert,
- mindestens einem Durchflusserzeuger (11) für die Schmierflüssigkeit (4), der es ermöglicht, mindestens einen Schmierkreislauf (2) mit der Schmierflüssigkeit (4) zu versorgen, und
- mindestens einer Vorrichtung (15-19) zum Auffangen von Partikeln,
**dadurch gekennzeichnet, dass** das Überwachungssystem (20) mindestens eine Partikelerfassungsvorrichtung (25), die in mindestens einem Schmierkreislauf (2) angeordnet ist, sowie mindestens einen Rechner (21) und eine Warnvorrichtung (22) umfasst, wobei das Überwachungssystem (20) konfiguriert ist, um das Verfahren nach einem der Ansprüche 1 bis 11 auszuführen.

13. Überwachungssystem (20) nach Anspruch 12,
**dadurch gekennzeichnet, dass** die mindestens eine Vorrichtung zum Auffangen von Partikeln (15-19) mindestens einen magnetischen Stopfen (19) oder mindestens einen elektromagnetischen magnetischen Stopfen (18) umfasst.

14. Überwachungssystem (20) nach einem der Ansprüche 12 bis 13,
**dadurch gekennzeichnet, dass** das Schmiersystem (10) mindestens eine Vorrichtung (13) umfasst, die geeignet ist, die Partikel einzufangen, wobei die Erfassungsvorrichtung (25) stromaufwärts von der mindestens einen Vorrichtung (13) angeordnet ist, die geeignet ist, die Partikel einzufangen.

15. Überwachungssystem (20) nach einem der Ansprüche 12 bis 14,
**dadurch gekennzeichnet, dass** mindestens eine Partikelerfassungsvorrichtung (25) induktiv, akustisch oder auch optisch ist.

16. Mechanisches System (1) mit:
- Drehelementen (6),
- Elementen (6) zur Kraftübertragung und zur Verringerung oder Erhöhung der Drehgeschwindigkeit, und
- einem Überwachungssystem (20),
**dadurch gekennzeichnet, dass** das Überwachungssystem (20) gemäß einem der Ansprüche 12 bis 15 ist.

17. Mechanisches System (1) nach Anspruch 16,
**dadurch gekennzeichnet, dass** das mechanische System (1) ein Kraftübertragungsgetriebe eines Luftfahrzeugs ist.

18. Luftfahrzeug mit einem mechanischen System (1),
**dadurch gekennzeichnet, dass** das mechanische System (1) nach einem der Ansprüche 16 bis 17 ist.

## Claims

1. Method for monitoring a mechanical system (1), said mechanical system (1) comprising a lubrication system (10) provided with:
- at least one tank (7) containing a lubrication liquid (4);
- at least one lubrication circuit (2), in which said lubrication liquid (4) circulates and which is intended for lubricating said mechanical system (1), said at least one lubrication circuit (2) comprising pipes (12), in which said lubrication liquid (4) circulates;
- at least one flow generator (11) for said lubrication liquid (4) for supplying at least one lubrication circuit (2) with said lubrication liquid (4); and
- at least one particle capture device (15-19),
**characterized in that** said method comprises the following steps:
- measuring at least one first feature of detected particles circulating in at least one pipe (12) of a lubrication circuit (2);
- comparing at least one first feature of said detected particles with a first threshold;
- collecting particles captured by said at least one capture device (15-19) if at least one first threshold is exceeded;
- measuring at least one second feature of said captured particles;
- comparing at least one second feature of said captured particles with a second threshold; and
- maintaining said mechanical system (1) if at least one second threshold is exceeded,
and, if said at least one second feature of said captured particles is less than said second threshold and at least one second feature from among said at least one second feature of said captured particles is greeater than a third threshold, with said third threshold being less than said second threshold, said method comprises additional steps of:
- analyzing said at least one first feature of said detected particles in order to identify whether said lubrication liquid is polluted;
- cleaning said mechanical system (1) if pollution of said lubrication liquid is identified; and
- scheduling regular captures of said particles captured by said at least one capture device (15-19) if no pollution of said lubrication liquid is identified.

2. Method according to Claim 1,
**characterized in that** a warning is triggered after a shutdown of said mechanical system (1) if at least one first threshold is exceeded.

3. Method according to any one of Claims 1 to 2, **characterized in that**, during said step of analyzing said at least one first feature, the presence of the pollution of said lubrication liquid is identified if a change over time of a first feature of said detected particles is an increasing curve with at least one change of slope, with said slope decreasing when the time increases.

4. Method according to any one of Claims 1 to 3,
**characterized in that**, during said step of analyzing said at least one first feature, the presence of the pollution of said lubrication liquid is identified if a change over time of a first feature of said detected particles comprises a first increase in a first time interval, then levelling-off in a second time interval, or even if said change includes several increases and several successive levelling-offs.

5. Method according to any one of Claims 1 to 3,
**characterized in that**, during said step of analyzing said at least one first feature, no pollution of said lubrication liquid is identified if a change over time of a first feature of said detected particles is constantly linearly increasing.

6. Method according to any one of Claims 1 to 3,
**characterized in that**, during said step of analyzing said at least one first feature, no pollution of said lubrication liquid is identified if a change over time of a first feature of said detected particles is an increasing curve with at least one change of slope, with said slope increasing when the time increases.

7. Method according to any one of Claims 1 to 6, **characterized in that**, when said mechanical system (1) equips an aircraft, said method comprises a step of transmitting said at least one first feature of said detected particles to a ground base in order to carry out said step of analyzing said at least one first feature.

8. Method according to any one of Claims 1 to 7,
**characterized in that** a first feature of said detected particles is to be selected from among the number, the size and the flow of said particles and a second feature of said captured particles is to be selected from among the number, the size, the mass, the material and the morphology of said particles.

9. Method according to any one of Claims 1 to 8, **characterized in that** said step of measuring at least one first feature of detected particles circulating in at least one pipe (12) of a lubrication circuit (2) is limited to detected metal particles.

10. Method according to any one of Claims 1 to 8, **characterized in that** said step of measuring at least one first feature of detected particles circulating in at least one pipe (12) of a lubrication circuit (2) is limited to the detected metal particles and said step of analyzing said at least one first feature of said detected particles for identifying whether said lubrication liquid is polluted is limited to the detected metal particles.

11. Method according to any one of Claims 1 to 10, **characterized in that** said step of collecting particles captured by said at least one capture device (15-19) if at least one first threshold is exceeded is limited to the captured metal particles.

12. Monitoring system (20) for monitoring a mechanical system (1), said monitoring system (20) comprising a lubrication system (10) for said mechanical system (1), said lubrication system (10) being provided with:
- at least one tank (7) containing a lubrication liquid (4);
- at least one lubrication circuit (2), in which said lubrication liquid (4) circulates and which is intended for lubricating said mechanical system (1), said at least one lubrication circuit (2) comprising pipes (12), in which said lubrication liquid (4) circulates;
- at least one flow generator (11) for said lubrication liquid (4) for supplying at least one lubrication circuit (2) with said lubrication liquid (4); and
- at least one particle capture device (15-19),
**characterized in that** said monitoring system (20) comprises at least one particle detection device (25) arranged in at least one lubrication circuit (2), as well as at least one computer (21) and a warning device (22), said monitoring system (20) being configured to implement the method according to any one of Claims 1 to 11.

13. Monitoring system (20) according to Claim 12, **characterized in that** said at least one particle capture device (15-19) comprises at least one magnetic plug (19) or at least one electric magnetic plug (18).

14. Monitoring system (20) according to any one of Claims 12 to 13,
**characterized in that** said lubrication system (10) comprises at least one device (13) capable of trapping some of said particles, said detection device (25) being arranged upstream of said at least one device (13) capable of trapping some of said particles.

15. Monitoring system (20) according to any one of Claims 12 to 14,
**characterized in that** at least one particle detection device (25) is inductive, acoustic or even optical.

16. Mechanical system (1) comprising:
- rotating elements (6);
- elements (6) for transmitting power and for reducing or increasing the speed of rotation; and
- a monitoring system (20),
**characterized in that** said monitoring system (20) is a monitoring system according to any one of Claims 12 to 15.

17. Mechanical system (1) according to Claim 16,
**characterized in that** said mechanical system (1) is a power transmission gearbox of an aircraft.

18. Aircraft comprising a mechanical system (1),
**characterized in that** said mechanical system (1) is a mechanical system according to any one of Claims 16 to 17.
